(19)

Europäisches
Patentamt
European
Patent Office
Office européen
des brevets

(11) **EP 4 006 543 A1**

(12) **EUROPEAN PATENT APPLICATION**

(43) Date of publication:
**01.06.2022 Bulletin 2022/22**

(21) Application number: **20209706.9**

(22) Date of filing: **25.11.2020**

(51) International Patent Classification (IPC):
***G01N 33/483*** (2006.01)

(52) Cooperative Patent Classification (CPC):
**G01N 33/4833**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **Lipsch, Job
  5656 AE Eindhoven (NL)**
• **van Gijsel, Thomas Johannes
  5656 AE Eindhoven (NL)**
• **Beckers, Lucas Johannes Anna Maria
  5656 AE Eindhoven (NL)**
• **Shulepov, Sergei Y.
  5656 AE Eindhoven (NL)**
• **Valster, Susanne Maaike
  5656 AE Eindhoven (NL)**

(74) Representative: **Philips Intellectual Property &
Standards
High Tech Campus 52
5656 AG Eindhoven (NL)**

(54) **DEVICES FOR CELL OR TISSUE CULTURING, OR TESTING IN VITRO MUSCLE TISSUE**

(57) A device for testing in vitro muscle tissue in a microwell plate comprises a base section and two pillars, each protruding from the base section. The device also comprises a wall section with an inner wall surface surrounding the pillars and an outer wall surface, wherein the wall section comprises at least two rib elements extending outwardly from the outer wall surface to an outer periphery of the device.

FIG.1

**Description**

FIELD OF THE INVENTION

The invention relates to devices for cell culturing, tissue culturing or devices for testing in vitro cells or tissue. In particular, the invention relates to devices for testing the reaction of in vitro muscle tissue to external electrical stimulation.

BACKGROUND OF THE INVENTION

In the field of drug research, many drugs are tested on cardiotoxicity. Many (new) drugs affect the heart muscle contraction and profile, thus giving toxic effects. Drug-induced cardiotoxicity is a major adverse effect that has been encountered for some clinically important drugs. This toxicity has previously led to the post-marketing withdrawal of numerous pharmacologically active drugs and limits the efficacy of other clinically useful ones. Almost 10% of drugs in the last four decades have been recalled from the clinical market worldwide due to cardiovascular safety concerns.

Drug-induced cardiotoxicity is an important cause of rejecting compounds in preclinical and clinical development. It represents one of the most serious side effects associated with novel drug development, and it is known to be one of the major toxic effects induced by several types of drugs. Assessing drug-induced cardiotoxicity risk including QT interval prolongation is considered nowadays an integral part of the standard preclinical evaluation of new chemical entities.

Cardiotoxicity may be tested in animal models, for example in rats. However, these models have drawbacks and do not always predict the effect in humans well. This may result in discarding potential beneficial medication, but also in accepting potentially toxic medication in a field trial on humans.

Another way to assess cardiotoxicity is to test the effect of the drug in vitro. The drug is added to an assembly of cardiomyocytes, which are grown in vitro. Afterwards, the effect to electrical stimuli is studied.

An example of such state of the art device is the HeartDyno (Trade Mark) device. This device consists of an oval shaped well, with two small pillars at the bottom. These devices are produced using thin film technologies. The mold is made via SU-8 photolithography on a wafer, resulting in 700 $\mu$m deep features. PDMS is cast over these features and cured. After removal of the PDMS from the wafer, samples of 6 mm diameter are punched.

These samples are for example placed inside the wells of a 96-well plate and glued to the bottom using silicone glue. A mixture of cardiomyocyte cells, cardio fibroblast cells, collagen, DMEM, NaOH and matrigel is added to the wells. The cardiomyocyte tissue is formed over several days. The tissue shows spontaneous contraction, but also contracts during electrical stimulation. During contraction, the two pillars deflect and the deflections are analyzed using video analysis algorithms.

SUMMARY OF THE INVENTION

Gluing the devices inside a well of a well plate is a cumbersome process. It is difficult to do this in a sterile way, and it requires extra manufacturing steps. Furthermore, the devices need to be aligned in such way that the pillars are oriented in the same way in each well, which requires extra care. A proposed solution is to 'clamp' the device inside the well of a well cell plate. This can be done by producing a device from an elastomeric material, with a slightly larger outer diameter than the well, so it will be kept in place by shear stresses. However, in this way, the negative space of the device will deform and this may render it unusable.

The invention is defined by the claims.

According to examples in accordance with an aspect of the invention, there is provided a device for testing in vitro muscle tissue, the device comprising:

> a base section;
> two pillars each protruding from the base section; and
> a wall section with an inner wall surface surrounding the pillars, and an outer wall surface, wherein the wall section comprises at least two rib elements extending outwardly from the outer wall surface to an outer periphery of the device.

In vitro cardiac muscle tissue can be electrically stimulated to contract in order to simulate the behavior of in vivo cardiac tissue. The tissue can be grown between and around the two pillars such that, when the tissue contracts, the two pillars deflect. The displacement of the pillars caused by the deflection can be measured to determine the force from the contraction of the tissue.

Typically, the devices for testing in vitro muscle tissue are put into a well of a microwell plate. In order to keep the device in the well, the outer periphery of the test unit can be made slightly larger than the diameter of the well such that the shear stresses keep the device in place. However, this could cause the inner diameter of the wall section to deform.

In order to avoid or reduce the inner wall surface deformation, rib elements are added to the wall section to avoid or reduce the inner wall surface from deforming whilst keeping the shear stresses high enough to keep the device in place during use of the device without the use of glue. The outer diameter of the rib elements (at the outer periphery of the device) may be larger than the outer diameter of the base section in order to further reduce the deformation of the device.

The pillars may be taller than the wall section or the wall section may be taller than the pillars. Alternatively, the pillars and the wall section have the same height. Similarly, the wall section may have the same height as the rib elements, or the rib elements may have a different height from the wall section (e.g. taller or shorter). Each rib element may have the same height or a different height.

There may be two, three, four or more rib elements extending in at least two non-parallel directions within a virtual plane comprising the base section. However in one preferred example, there are four rib elements. A symmetrical design has the rib elements extending radially at 90 degrees apart from each other.

However, an asymmetrical design may be used with two pairs of diametrically opposite rib elements, but with the two pairs not orthogonal to each other (so making a non-perpendicular cross).

This forms a cross shape of the rib elements to provide a structure which would not deform under shear stress from any direction.

The inner wall surface may be elliptic or even rectangular, hence a shape with a non-unity aspect ratio.

This non-unity aspect ratio encourages the tissue to grow in a contraction direction in line with the separation gap between the pillars.

The contraction direction is the direction in which the tissue contracts. Thus, by having the contraction direction in line with the spacing between the pillars, the tissue will deflect the pillars towards the center point between the two pillars.

The height of the rib elements may be at most the same height as the height of the wall section. The height of the wall section may be at most 2000μm.

The height of the pillars may be at least 500μm and at most 2000μm.

The device may be manufactured based on:

     injection molding;
     injection compression;
     transfer molding; or
     embossing.

The pillars are bulk manufactured using on of these methods in order to reduce the spread in pillar stiffness from the manufacturing method and increase the production rate, when compared to hand casting.

The device (or only the pillars, the base section and/or the rib elements) may be manufactured from a silicone rubber. The silicone rubber may comprise Elastosil 3040/30.

The pillars may be tapered towards the distal end, and the distal end of the pillars may be rounded or partly rounded.

Tapering the pillars allows the demolding step (of the manufacturing process) to be more reliable. Similarly, a rounded distal end of the pillars further increases reliability in the demolding step.

The Young's Modulus of the material used to manufacture the pillars may be at least 0.5 MPa and at most 5 MPa. For example, the Young's Modulus is between 1 MPa and 2 MPa. The stiffness of the pillars will depend on the Young's Modulus and the geometry of the pillars.

However, there are other material properties which are also relevant for the function of the pillars. The tensile strength (e.g. 9.0 N/mm$^2$) and the max elongation (e.g. at break 600%) are two of such relevant properties. The combination of the these properties makes it possible to demold the pillar structures with an aspect ratio near 5 (or even higher) and also for allows the demolding of mushroom geometries.

Thus, using injectable molding grades of silicone gives reliable end properties after manufacturing with easy scale up possibilities and is also suitable for geometries with an aspect ratio of 5 or higher for releasing from industrial molds. This is not possible with other casting solutions.

The invention also provides a set of devices for testing in vitro muscle tissue in a microwell plate wherein the set of devices is manufactured using multi cavity injection molding, wherein each cavity corresponds to one device.

Multi injection molding would increase the production rate of the devices, as well as ensuring an adequate mold flow and wall thickness ratio.

The invention also provides a system for testing in vitro muscle tissue, the system comprising:

     an array base;
     an array of devices for testing in vitro muscle tissue in a microwell plate on the array base, wherein the array base and the array of devices are formed as a single unit.

For example, the devices could be equally spaced on the array base in a rectangular array. This allows multiple devices to be manufactured without the need of a multi cavity injection molder.

The system may further comprise a microwell plate with a plurality of wells, wherein the plurality of devices on the array base are situated such that each device fits within a respective one of the wells.

For example, using an open bottom microwell plate, the system with the array of devices could be placed under the microwell plate such that the devices all fit within the wells of the microwell plate. The system would be manufactured to fit within the microwell plate (i.e. the outer diameter of the devices would fit within the wells and the spacing of the devices would be the same as the spacing between the wells).

The muscle tissue can then be grown in the well of the microwell plate around the pillars of the device and within the wall section.

The devices may be manufactured of elastic material and the outer periphery of each device may be larger than the inner periphery of the wells of the microwell plate by, at most, 5%.

If the devices have a slightly larger size than the wells and are manufactured from a flexible material, the devices will be able to fit within the well, but will provide a shear stress to the device due to the difference in diameter. The difference in diameter is dependent on the material used to manufacture the devices, the shear stress required to keep the device in the well during muscle contractions etc.

The system may further comprise a pair of electrodes for stimulating muscle tissue.

The invention also provides a method of manufacturing a device for testing in vitro muscle tissue, the method comprising:

injection molding the device using a mold which defines:

a base section;
two pillars each protruding from the base section; and
a wall section with an inner wall surface surrounding the pillars, and an outer wall surface, wherein the wall section comprises at least two rib elements extending outwardly from the outer wall surface to an outer periphery of the device.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a representation of a device with two tapered pillars;
Fig. 2 shows model pillars for calculating the displacement of the tapered pillars due to forces applied;
Fig. 3 shows simulated pillars with tapered and rounded tops;
Fig. 4 shows a 3D representation of the device with pillars;
Fig. 5 shows a top down view of a device manufactured with injection molding;
Fig. 6 shows an injection molded device with cardiac muscle tissue;
Fig. 7 shows the results of a first experiment;
Fig. 8 shows the results of a second experiment;
Fig. 9 shows a top view of a device;
Fig. 10 shows a device with rib elements around the outer perimeter of the device;
Fig. 11 shows a device with rib elements manufactured with injection molding;
Fig. 12 shows an array of devices for integration in a well cell plate;
Fig. 13 shows a 96 well cell plate with an open bottom;
Fig. 14 shows a plurality of devices manufactured with multi cavity injection molding;

Fig. 15 shows an inverted pillar device;
Fig. 16 shows a device with a staircase like structure for determining the height positioning of the muscle tissue;
Fig. 17 shows a plan view of the device of Fig. 16; and
Fig. 18 shows a device with a conical structure for the height determination.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for testing in vitro muscle tissue, for example in a microwell plate, the device comprising a base section and two pillars, each protruding from the base section. The device also comprises a wall section with an inner wall surface surrounding the pillars and an outer wall surface, wherein the wall section comprises at least two rib elements extending outwardly from the outer wall surface to an outer periphery of the device.

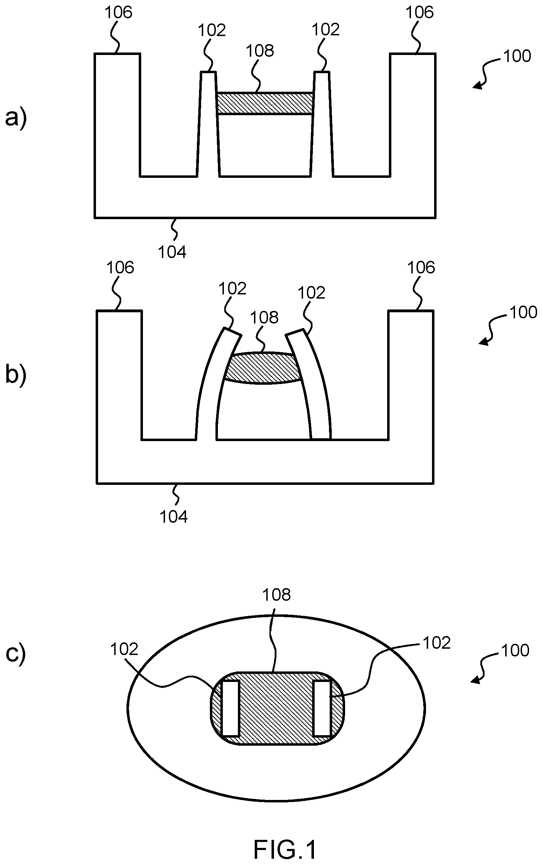

Fig. 1 shows a representation of a device 100 with two tapered pillars 102. The pillars 102 are made of flexible material, the shape of the pillar 102 is designed for mass production and the shape and height of the pillars 102 are adjusted to have the required material properties for testing samples of tissue 108. The design of the pillars 102 is adapted to ensure demolding. The pillars 102 are tapered, with a wider base and smaller top.

The pillars 102 extend from a base section 104 and are enclosed within the inner surface of a wall section 106. The pillars 102 are designed such that cardiac tissue 108 can grow between and around the pillars 102. The cardiac tissue 108 can then be contracted with electrodes and the contractions of the cardiac tissue 108 from the electrical signal can be measured by the displacement of the pillars 102.

Fig. 1 a) shows a cross section of the device 100 with cardiac tissue 108 between the pillars 102 with no contraction. Fig. 1 b) shows the same device 100 but with the cardiac tissue 108 contracting. The pillars 102 are displaced due to the contractions of the cardiac tissue 108.

Fig. 1 c) shows the same device 100 from a top down view when the cardiac tissue 108 has not contracted. In this example, the inner wall surface is elliptical. By growing cardiac cells in the ellipsoid cavity the tissue 108 forms a direction for contraction in line with the spacing between the pillars 102.

The device 100 could be manufactured by injection molding, 3D printing, urethane casting or press casting. The tapered pillars 102 allow a more robust demolding method for injection molding, urethane casting and press casting and also allow a more stable structure to be printed from 3D printing methods. These methods allow mass production of the devices 100. The following examples will be described in connection with injection molding. However, any feature or advantage disclosed may also apply to other manufacturing methods.

In this example the base section is shown at the bottom. However, this is not always the case and the base section may have any orientation.

The pillars 102 may have a cuboid shape and be tapered on one, two, three or four of their sides. For example, two sides of the pillars 102 (e.g. facing inwardly towards the tissue and facing outwardly away from the tissue) may be tapered and the other two sides may be perpendicular to the base section 104. The exact taper for a pillar 102 will depend on the dimensions of the pillar 102 itself but may range from a 1 degree taper to a 20 degree taper relative a perpendicular line from the base section 104.

The pillars 102 may instead be cylindrical and as such the one side would be tapered. Similarly, the exact taper for a pillar 102 will depend on the dimensions of the pillar 102 itself but may range from a 1 degree taper to a 20 degree taper relative to a perpendicular line from the base section 104. An example taper would be between 0.5 degrees and 2 degrees.

Additionally, the distal end of the tapered pillars 102 from the base section (i.e. the tip of the tapered pillars) may also have a head section. The pillar head would have a larger diameter (in one or two dimensions) than the rest of the pillar 102 and would stop tissue from sliding towards the distal end of the pillars and disconnecting itself from the pillars.

For a device 100 with a head section which is injection molded, the pillars 102 (beneath the head) are again preferably tapered. The tapering of the pillars reduces the friction between the pillars 102 and the mold when demolding the devices 100. The tensile strength (e.g. 7 $N/mm^2$ to 11$N/mm_2$, preferably 9.0 $N/mm^2$) and the maximum elongation (e.g. at break 400% to 800%, preferably 600%) of the material (i.e. silicone rubber) used to manufacture the pillars make it possible to demold the pillar structures with a head section.

Due to the reduced friction between the tapered pillars 102 and the mold, the only significant force required to remove tapered pillars 102 with head sections is the force required to elastically deform the pillar head to fit through the smallest section of the mold. The properties (e.g. tensile strength and maximum elongation) of the silicone rubber allow it to deform elastically in such a manner and thus retain the original shape once it has been removed from the mold. Other materials with similar properties could also be used.

The shape of the head section can also be designed to reduce the force required to pull it out of the mold. The shape of the head section will also depend on the properties of the material, the shape of the pillars 102 and the taper used on the pillar. For example, the head section could be a rounded shape, for example even spherical with a diameter larger than (or equal to) the width of the largest side of the pillars 102. The pillars with a head section could also have a mushroom-like geometry.

Thus, using injectable molding grades of silicone gives reliable end properties after manufacturing with easy scale up possibilities and is also suitable for tapered pillars 102 with a head section. This is not possible with other casting solutions.

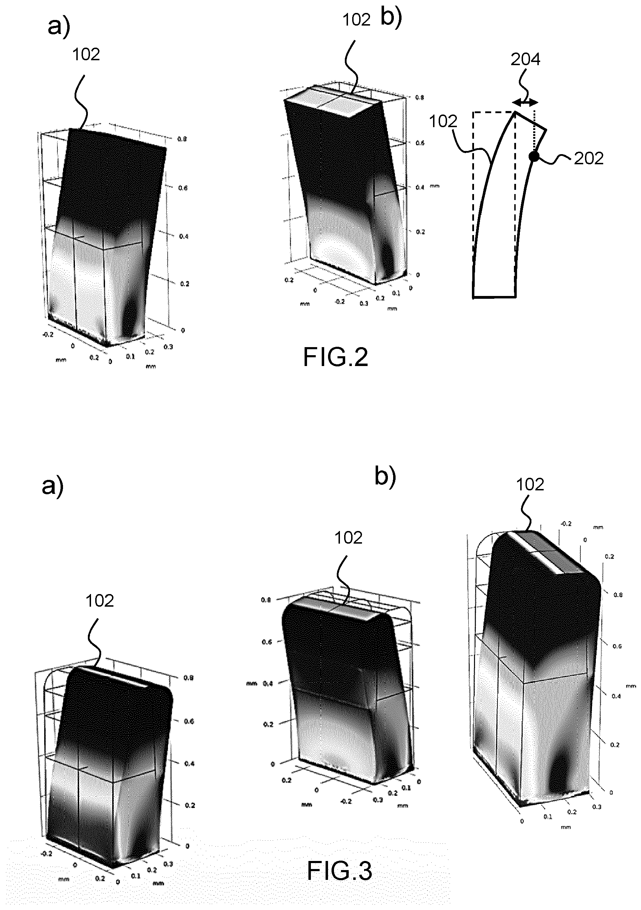

Fig. 2 shows model pillars 102 for calculating the displacement of the tapered pillars 102 due to forces applied. The tapering of the pillars 102 influences the stiffness and elasticity of the pillar 102 and this should be compensated. In translating the design into an injection molding design, changes in material and geometry should be compensated to keep correct stiffness and elasticity of the pillars 102. The influence of changing the design of the pillars 102 design can be calculated with simulation techniques.

A common soft material for injection molding is Elastosil 3040/30, with shore hardness 40A. Producing the device 100 with this material influences the elasticity characteristics of the pillar 102. Simulations have been performed to estimate these effects, and to propose an optimized design.

Using injection molding increases the reproducibility of the stiffness, and the hardness is much better. The silicone from Sylgard 184 is not so reliable due to temperature variations of the curing, in combination with poor accuracy of the mixing (and hence weight differences) of the two components in a desired, e.g. 9:1, ratio for the silicone A and the cross linker B. For injection molding, components A and B are mixed in a 1:1 ratio in much larger quantities and the stoichiometric mismatch is minimal. It can also be scaled up easily.

After injection molding, the reaction of the Elastosil 3040/30 will initiate after heating up in the mold to 150C or higher. A problem with the Sylgard 184 is that it starts to cure at room temperature. The idle time is also low for Sylgard 184 so that it becomes stiff sooner, such that casting it in small cavities may not be possible. With injection molding, the mixture heats up in the heated mold and has a low viscosity at high pressure (i.e. 30MPa to 60 MPa), such that all the cavities for the pillars 102 will be filled.

Fig. 2 a) shows a simulation of tapered pillars 102 for measuring the strain on the pillars 102 when a force is applied. The typical elastic modulus of Sylgard 184 is 1.45 MPa and for Elastosil is 1.4 MPa. Bulk modulus of 2.2 GPa is used for both materials. A Neo-Hookian approximation is employed in the calculations.

Fig. 2 b) shows a simplified representation of the displacement measurement 204. The measurement point 202 on the pillars 102 is at ¾ height. Devices using Slygard 184 have a stiffness of 500 um/N at 100 $\mu$m displacement, so the stiffness of the Elastosil pillars 102 with respect to the Sylgard 184 pillars is estimated at 100 $\mu$m displacement.

In the simulation of Fig. 2 a), a distributed force is applied at the defined area of a pillar 102 and an area between ½ and ¾ of the pillar 102 height is subjected to this force. The force is increased until the horizontal displacement of the monitoring point (at ¾ height of the pillar 102) reaches 100 $\mu$m. The pillar 102 heights are 800 $\mu$m.

The stiffness of the Sylgard 184 pillars is derived at 100 $\mu$m displacement, and compared to the stiffness of the Elastosil 3040/30 pillars 102. The stiffness of the tapered pillar design is about 2 times higher than the Slygard 184 pillars. This can be addressed with a new pillar shape (i.e. higher pillar 102, different tapering angle, etc.). The choice of stiffness will depend on the needs of any particular user.

For example, higher pillars 102 can be used to compensate for the changed stiffness so that the pillars 102 can be produced with injection molding techniques, making the design suitable for mass production.

Fig. 3 shows simulated pillars 102 with tapered and rounded tops. Fig. 3 a) shows a pair of pillars 102 with a height of 800 $\mu$m. Fig. 3 b) shows a pillar 102 with a height of 900 $\mu$m.

The rounded top design is 15% stiffer than the reference configuration shown in Fig. 2. However, because this can be mass manufactured, the spread in stiffness will be much lower. To compensate completely for the stiffness change, pillars 102 of 950-970 $\mu$m height have been found to be suitable. The rounded top pillars 102 are better able to distribute local stresses. The rectangular pillars 102 have a higher stress concentration which gives higher variations.

A mold for a device 100 with tapered and rounded tops has been designed and made, and devices 100 have been made from this mold. Demolding of 900 $\mu$m pillars 102 in Elastosil 3040/30 material was found to be successful.

The mold has a base portion for defining an outer wall (the underside) of the base section 104 and an outer wall (the radially outward facing outer wall) of the wall section 106. A top portion is for defining an inner wall (the top side) of the base section 104, the inner wall (the radially inwardly facing wall) of the wall section 106 and the pillars 102, wherein the top portion comprises two cavities for forming the two pillars 102.

The cavities were fitted with an insert to define the pillars, so the shape can easily be changed to answer varying product requirements. Furthermore, this way of production ensures a well defined top shape of the pillars 102. Different inserts could be used in the cavities to produce different pillar shapes (and thus different stiffness values).

By growing cells in the ellipsoid cavity, the muscle tissue 108 forms a direction for contraction in line with the spacing between the pillars 102 (i.e. parallel to a line joining the centers of the two pillars), but initially the cells may not attach to the pillars 102. Uniform cell proliferation on the extracellular matrix in this device 100 into a confluent layer of cells to form muscle tissue 108 is a critical step.

To solve this, silicone pillars 102 can be modified with carboxylic groups so that direct cell attachments to the pillars 102 is possible. By bulk modification of silicone with linoleic acid, carboxylic groups are introduced and active on the surface so that cell can adhere onto the pillars 102. In this way cardiomyocytes can better grow into muscle tissue 108 between the two pillars 102.

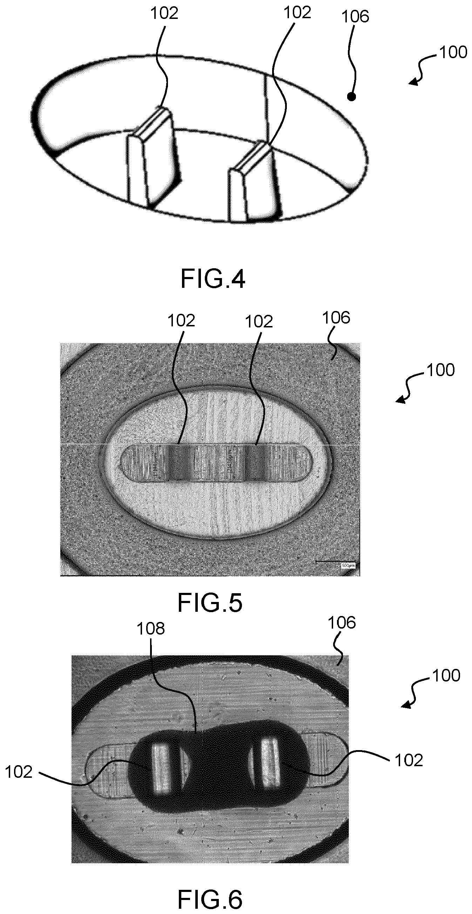

Fig. 4 shows a 3D representation of the device 100 with pillars 102. In this example, the inner surface of the wall section 106 has an elliptical shape. The device 100 can be described as having a cavity with two pillars 102 within the cavity (i.e. the cavity is defined by the wall section 106).

Additionally, a pillar head could be added to the distal (top) end of the pillars 102 to avoid the muscle tissue 108 from disconnecting from the pillars 102 due to the movement of the muscle tissue 108 caused by the contractions. The elasticity of the material will allow the pillars 102 to be demolding despite the pillar head if suitable dimensions are chosen.

Fig. 5 shows a top down view of a device 100 manufactured with injection molding. Marking from the injection molding process can be seen near and around the pillars 102. Similarly, in this example the inner surface of the wall surface 106 is elliptical.

Fig. 6 shows a device 100 with cardiac muscle tissue 108. The cavity was filled with gel (Extra Cellular Matrix) and the cardiomyocytes grew on top of the gel into muscle tissue 108. When the tissue 108 contracts, the pillars 102 will move and the muscle function can be monitored when the tops of the pillars 102 are provided with suitable indicators (e.g. colors applied to the top of the pillars to enable more precise focusing of a microscope).

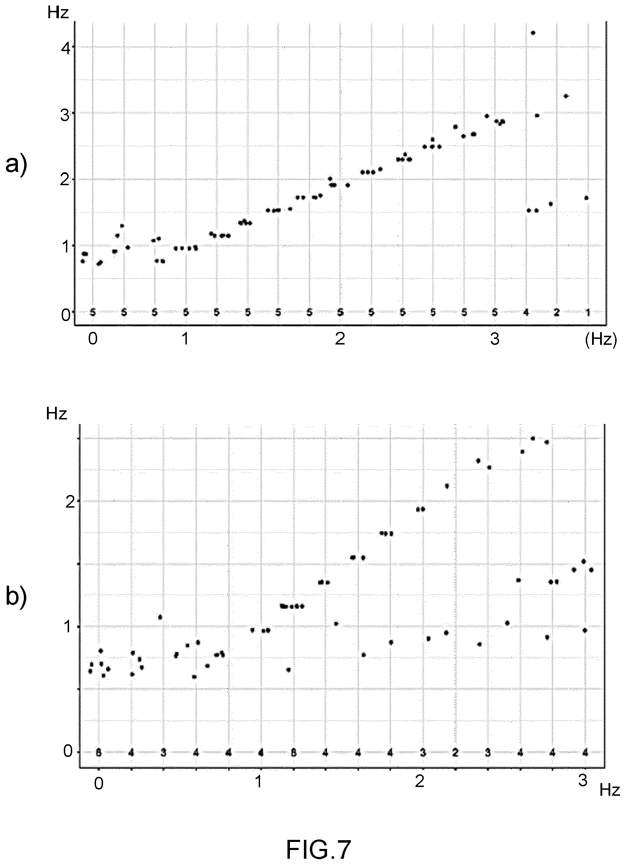

Fig. 7 shows the results for a first experiment. The graph shows a plot of beating frequency (y-axis) versus pacing (stimulation) frequency. The pacing frequency is the frequency of the electrical signal used to stimulate the muscle tissue 108 and the beating frequency is the frequency at which the muscle tissue 108 is observed to contract.

The experiment involved stimulating cardiac muscle tissue 108 in the device 100 and comparing the result to other devices. The formed tissue 108 was electrically stimulated with 35V, a pulse duration of 10 ms and a range of pacing frequencies with increments of 0.2 Hz. The muscle contraction force was calculated using the following equation:

$$F = \left(\frac{Ebh^3}{2(-x^3 + 3Lx^2)}\right)\delta$$

Equation 1: where: F = Force, E = Youngs' Modulus, b = pillar length, h = pillar width, x = position of tissue on pillar in z-direction, L = height of the pillar, $\delta$ = displacement.

The results obtained are shown in Figs. 7 a) and 7 b). Fig. 7 a) shows the beating frequency (y axis) as function of the pacing frequency (x axis) for five tapered pillar devices 100 manufactured with injection molding. Fig. 7 b) shows the beating frequency (y axis) as function of the pacing frequency (x axis) on five HeartDyno devices.

It is observed that the tapered devices are on a par with the HeartDyno devices or perform even better due to the cardiomyocyte tissue 108 responding more consistently to pacing at a higher frequency. The tapered devices used for the results in Fig. 7 a) were also rounded.

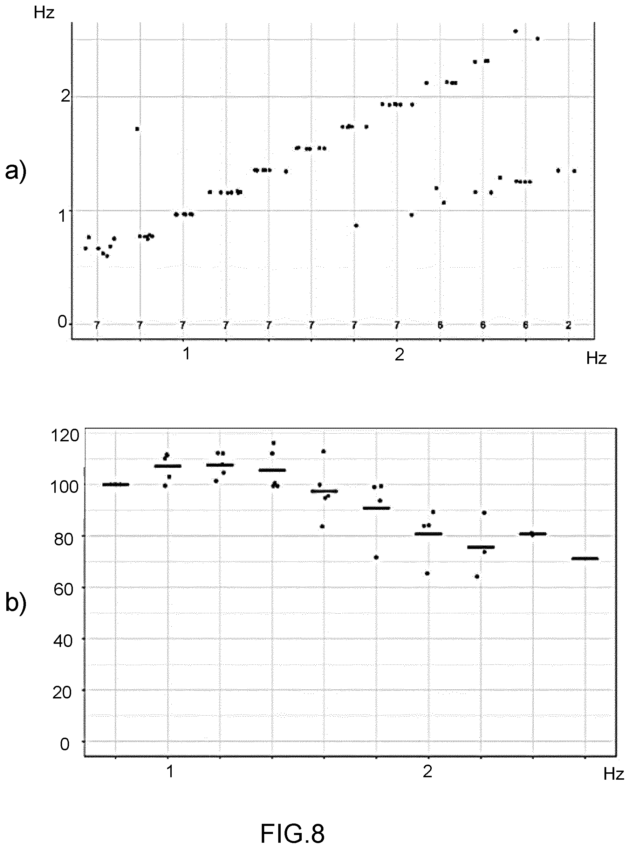

Fig. 8 shows the results of a second experiment. The experiment again involved stimulating cardiac muscle tissue 108 in the device 100. Fig. 8 a) shows the beating frequency (y axis) as function of the pacing frequency (x axis) of seven tapered pillar devices 100. Fig. 8 b) shows the normalized beating frequency (y axis) against the pacing frequency (x axis) for the seven tapered pillar devices 100. It can be seen that the contraction amplitude goes up, which indicates a positive force-frequency relationship. This is a hallmark of mature cardiomyocytes.

These results show that the tapered pillar devices 100 perform on a par or slightly better than the HeartDyno devices. Furthermore they have the advantage that the stiffness spread is lower due to the use of mass production techniques. Also the cost can be lower due to the use of mass production techniques.

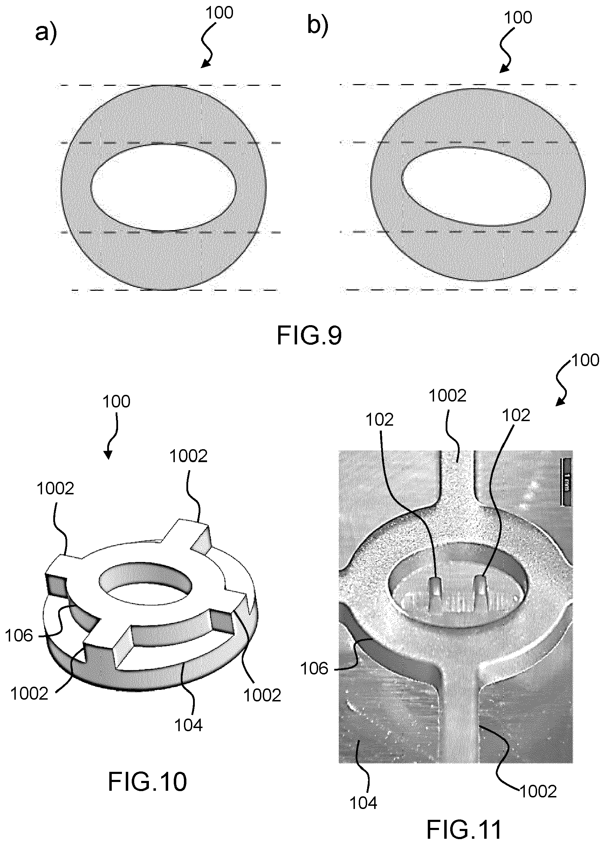

Fig. 9 shows a top view of a device 100 (pillars 102 not shown). Fig. 9 a) shows a device 100 inserted in the well of a microwell plate (i.e. well cell plate) adequately. Fig. 9 b) shows a device 100 when it is deformed due to being inserted in the well of a well cell plate inadequately. This deformation can occur if the device is made slightly larger than a well in a well cell plate (to avoid using glue).

The deformation is exaggerated in Fig. 9 b) and may be caused after the device 100 is positioned in a non-ideal well shape causing unpredictable deformation of the inner surface and thus influencing part to part differences in cell growth performances and reproducibility.

Fig. 10 shows a device 100 with base section 104, wall section 106 and rib elements 1002 around the outer perimeter of the device 100 (pillars 102 not shown). This makes it possible to use a slightly larger outer diameter of the device 100, compared to the inner surface of the well of a well cell plate, while not deforming the inner surface of the device 100. In this way, the shear stress of the device 100 against a well cell plate can be increased to such a point that gluing the device 100 in the well cell plate is no longer necessary. Thus, the rib elements 1002 (in this case, cross ribs) can increase the shear stress and reduce deformation of the device 100, in particular, deformation of the inner surface.

The device 100 is made of an elastomeric material, which can be deformed upon application of an external force. Designing cross ribs 1002 around this wall section 106 stops the inner wall surface from deforming when inserted in a well, while keeping the shear forces high enough to avoid any form of adhesive needed between the device 100 and the well.

Additional benefits besides eliminating the necessity to use glue include that the flexible material of device 100 and integrated cross ribs 1002 will overcome tolerance issues of the shape of the device 100 or tolerances of an array (part to part variations are overcome). In some manufacturing methods, the demolding direction of either the device 100 and/or the well cell plate can help to increase shear forces or provide a complete mechanical lock.

In this example, devices 100 with four cross ribs 1002 are shown. However, the devices 100 could also have two cross ribs 1002, three cross ribs 1002 or more than four cross ribs 1002.

Fig. 11 shows a device 100 with rib elements 1002 manufactured with injection molding. The device 100 is made via injection molding of Wacker Elastosil LR 3040/40 (shore hardness A 40). The device 100 is punched with a 6.4-mm-punch and can be placed in a well of a well cell plate (e.g. with 96 wells), with an inner diameter of 6.4 mm

It has been found that the shear stress of the devices 100 inside the well of the well cell plate is high enough to keep the devices 100 in place, during a complete protocol of growing and testing cardiac tissue with a duration up to 9 days.

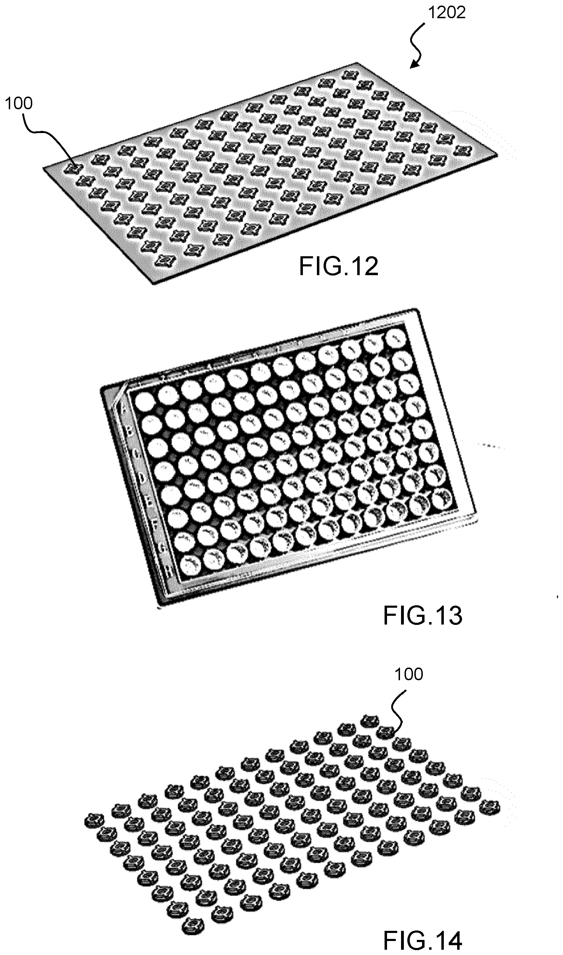

Fig. 12 shows an array 1202 of devices 100 for integration in a well cell plate. Besides single devices 100, the devices 100 can also be produced as an array 1202 integrated on a slab of material. For this purpose, a slab of material is produced, with an array of devices 100 with cross ribs 1002. This slab can be placed under a well cell plate with an open bottom. In this way, the array 1202 closes the well cell plate and provides the devices 100 for cell culture. The array 1202 can be circular, rectangular or any other ratio in between depending on the requirements.

Fig. 13 shows a 96 well cell plate with an open bottom. This well cell plate can be used for the array 1202 of devices shown in Fig. 12. Alternatively, devices 100 in an array 1202 integrated onto a material slab can also be directly used without the need of a well cell plate. In this case, the outer walls define the devices 100 of the array 1202 directly over a shared base, and this structure can be produced in one piece. The outer walls will act as an alternative to the wells in the well cell plate.

Fig. 14 shows a plurality of devices 100 manufactured with multi cavity injection molding.

Producing one slab material (devices in an array 1202) requires a big injection molding machine to fill the large volume and the small details of the pillars 102. Injection molding works best with even wall thicknesses (e.g. thickness of the wall and of the pillars 102). If they differ by a large ratio, small details will freeze more rapidly even when the mold has not been filled completely and large volumes will freeze by polymerization the slowest. An unwanted situation may occur if the smallest details are frozen already while the bigger volumes still need to be filled. This will cause defects and, in this case, the risk that the mold part for the pillars 102 is not filled completely.

This issue makes it difficult to produce the small details (i.e. the pillars 102) in the large slab. This can be solved by balancing the amount of material and wall thickness. Fig. 14 shows several separate devices 100 each with an optimized wall thickness and flow path. Less volume to freeze also improves production cycle times.

By manufacturing separate devices 100, tolerances differences within well cell plates can be overcome compared to an array 1202 of devices 100 with pillar details. Producing an array 1202 may lead to leakage in the well plate due to tolerance and shrinkage differences whereas producing separate devices 100 can overcome this. The devices may be manually or robotically positioned into e.g. a 96-well cell plate or any other target.

One problem that may be encountered with these devices 100 is knowing the exact height of the muscle tissue 108 on the pillars 102. As shown before (in equation 1), the displacement of a pillar 102 is dependent on the height of the muscle tissue 108 on the pillar 102. Also, this position can change during experiments. The muscle tissue 108 is known to creep up the pillars 102 due to mechanical forces. Furthermore, during pillar deflection, the muscle tissue 102 can come off of the pillars 102, rendering the experiment unsuccessful.

In order to avoid this problem, the device 100 may be "inverted", such that the pillars 102 protrude downwards (relative to a well cell plate).

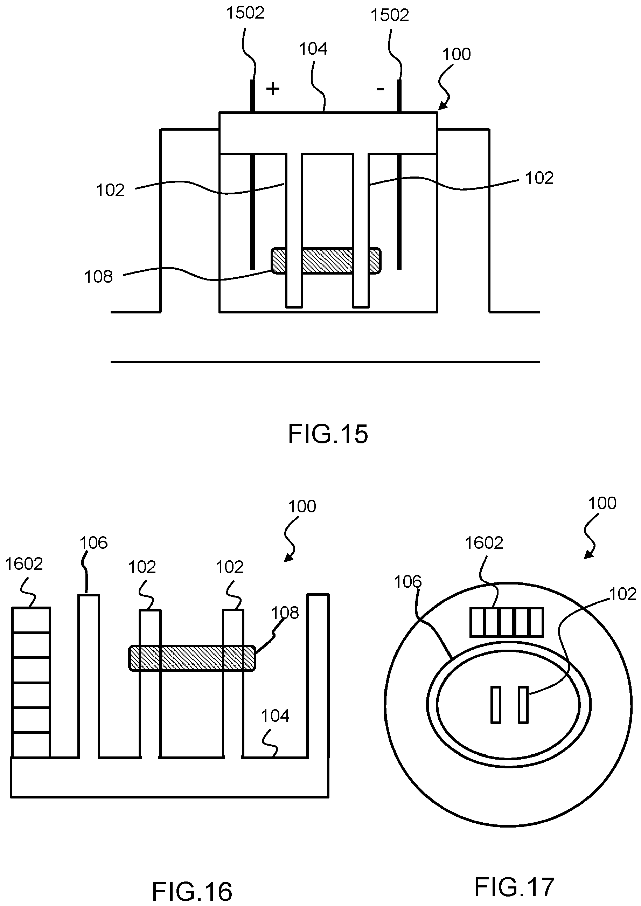

Fig. 15 shows an inverted pillar device. The inverted pillar device has two pillars 102 protruding from the base section 104. The inverter pillar device is fitted on a so-called ultra-low attachment well in a well cell plate. The pillars 'hang' in the well and muscle tissue 108 can be grown around them. Pillar 102 movement analysis can take place from below the well (if the well call plate is made of a transparent material).

A cell/collagen mixture is deposited on the bottom of the ultra-low attachment well. The base section 104 is placed on top of the well and acts as a lid for the well while the pillars 102 project into the cell/collagen mixture. When the cardiomyocyte tissue 108 forms, it grows around the pillars 102. It will not adhere to the well, because an ultra-low attachment well is used. When the cells start to contact each other, the pillars 102 will begin to move. This motion is captured with a microscope placed below the well.

In the inverted pillar device, the cell culture is always at the end of the pillars 102, thus the height position of the muscle tissue 108 is well defined. This makes the forces required for deflecting the pillars 102 more reproducible. Also, upon movement of the pillars 102, the muscle tissue cannot detach itself from the pillars 102 due to gravitational forces. Furthermore, the relative position of the two pillars 102 is defined and will not add additional spread.

An additional advantage of this example is that the inverted pillar device can again be mass produced via injection molding. Also, the range of possible material stiffness values is larger, because the pillars 102 hang from the base section 104. In an upright device 100 design, the pillars 102 need a certain stiffness to keep standing straight.

The measurement system may comprise a microscope underneath the well cell plate looking up. This is comparable to current systems for measuring pillar displacement. In either system, putting something on the bottom of the well will interfere with the measurements.

In this inverted design, the base section can functions as a plug for the well, avoiding spill or sealing the well from contaminants (e.g. air).

Cardiomyocyte muscle tissue 108 can be paced as mentioned above. This is implemented using a voltage of, for example, 35 V at a certain frequency (e.g. simulating heart beats). The cardiomyocytes will contract upon stimulation. This contraction can be monitored, while adapting the pace frequency. In order to pace the cells, two electrodes 1502 are placed in the well.

These electrodes can be integrated in the inverted device as shown in Fig. 15. In this way, positioning the electrodes does not hamper the field of view for cell visualization from the bottom of the well. Furthermore, the electrodes 1502 are always positioned adequately as they can be held by the base section 104 and the well does not have to be opened (i.e. the base section 104 does not have to be removed) in order to stimulate the muscle tissue 108. This prevents contamination of the cell culture.

For the conventional upwardly projecting pillars, it is of interest to be able to determine the height of the muscle tissue 108 up the pillars.

Fig. 16 shows a device 100 with a staircase like structure 1602 located outside the cavity area. The staircase structure 1602 is used to determine the height positioning of the muscle tissue 1602 on the pillars 102. The staircase in one example has steps of 50 $\mu$m each, but this dimension can of course be changed depending on the size of the device 100, the pillars 102 and/or the cell well plate. For example, if the device 100 with a structure 1602 is manufactured using injection molding, the step size can be changed by using a different injection molding insert.

When using a microscope to view the muscle tissue 108, the microscope must be focused on the muscle tissue 108 itself. When focusing on the muscle tissue 108 on the pillars 102, the z-position (i.e. height) can be determined by looking at the corresponding steps of the staircase structure 1602 which are also in focus. Each step of the staircase structure 1602 can be marked (e.g. with numbers or letters or other characteristic feature) to easily read out the corresponding z-position. The height of the muscle tissue 108 can then be determined and thus the forces on the pillars 102 can be normalized through calculation (i.e. using equation 1).

In this example, the wall section 106 has a separate outer surface for the structure 1602 outside the outer wall which defines the cavity. The structure 1602 can instead be placed within the inner surface of the outer wall 106. The height determination structure may then be on the inside of the wall 106 close to the pillars so easily visible.

Fig. 17 shows a plan view of the device of Fig. 16.

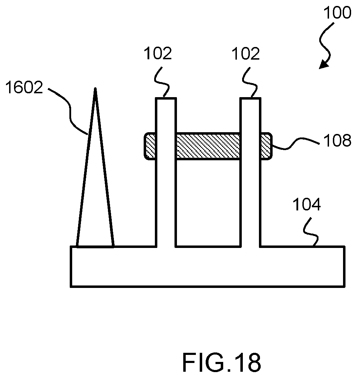

Fig. 18 shows a device 100 with a conical structure 1602 for the height determination. In this example, the diameter of the cone changes with the height of the structure 1602. Thus, the diameter of the cross section of this cone which is in focus when using a microscope corresponds to the z position of the muscle tissue 108. In this example there are no discrete steps in measurements of height (as experienced with the staircase structure) and as such the height can be measured at any position.

The structure 1602 could be any other shape in which the cross section of the structure 1602, when viewed from a top down view, changes relative to the height of the structure 1602 from the base section 104 (e.g. tapered 3D shapes, step based shapes etc.).

The structure 1602 may be taller than the pillars 102 and/or the wall section 106 or it may be the same height as either of them. In the case that the structure 1602 is taller than the pillars 102, the structure 1602 could have a height mark on it which would indicate the height of the pillars 102 (or a height slightly shorter than the height of the pillars) such that it can act as a warning mark for the user of a microscope to be aware that, if the muscle tissue 108 and the height mark are both in focus, the muscle tissue 108 may be in danger of disconnecting from the pillars 102.

Alternatively, the structure 1602 may be shorter than the pillars 102. In this case, once the user of a microscope can no longer focus on the structure 1602, this may act as a warning sign that the muscle tissue 108 may be in danger of disconnecting from the pillars 102. There may also be a plurality of height marks on the structure 1602 at different height locations to make the height measurements easier.

The previous examples have been described using a microscope to image the pillars 102 and thus detect and measure the displacement of the pillars 102. However, any other imaging system capable of measuring the pillars 102 may also be used (e.g. cameras etc.). Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

**Claims**

1. A device (100) for testing in vitro muscle tissue (108), the device (100) comprising:

   a base section (104);
   two pillars (102) each protruding from the base section (104); and
   a wall section (106) with an inner wall surface surrounding the pillars (102), and an outer wall surface, wherein the wall section (106) comprises at least two rib elements (1002) extending outwardly from the outer wall surface to an outer periphery of the device (100).

2. The device (100) of claim 1, wherein the wall section (106) comprises at least three rib elements (1002) extending in at least two non-parallel directions within a virtual plane comprising the base section, for example four rib elements extending radially at 90 degrees apart from each other.

3. The device (100) of claim 1 or 2, wherein the inner wall surface has a non-unity aspect ratio, for example the inner wall surface is elliptic or rectangular.

4. The device of any one of claims 1 to 3, wherein the height of the rib elements (1002) is at most the same height as the height of the wall section (106) and wherein the height of the wall section (106) is at most $2000\mu$m.

5. The device (100) of any one of claims 1 to 4, wherein the height of the pillars (102) is at least $500\mu$m and at most $2000\mu$m.

6. The device (100) of any one of claims 1 to 5, wherein the Young's Modulus of the material used to manufacture the pillars (102) is at least 0.5 MPa and at most 5 MPa.

7. The device (100) of any one of claims 1 to 6, wherein the device (100) is obtainable by any one of the following methods:

> injection molding;
> injection compression;
> transfer molding; or
> embossing,
> and preferably by injection molding.

8. The device (100) of any one of the previous claims, wherein the device (100) or pillars, and preferably also the base wall section and ribs comprise or consist of a silicone rubber.

9. The device (100) of claim 7 or 8, wherein the pillars (102) are tapered towards the distal end with respect to the base section (104), and the distal end of the pillars (102) are rounded or partly rounded at the interface between one or more sides walls of the pillar and the distal end of the pillar (102).

10. A set of devices (100), each as claimed in any one of claims 7 to 9, wherein the set of devices (100) is manufactured using multi cavity injection molding, wherein each cavity corresponds to one device (100).

11. A system for testing in vitro muscle tissue, the system comprising:

> an array base;
> an array (1202) of devices (100) each as claimed in any one of claims 7 to 9 on the array base, wherein the array base and the array (1202) of devices (100) are formed as a single unit.

12. The system of claim 11, further comprising a microwell plate with a plurality of wells, wherein the array (1202) of devices (100) on the array base are situated such that each device fits within a respective one of the wells.

13. The system of claim 12, wherein the devices (100) are manufactured of elastic material and wherein the outer periphery of each device (100) is larger than the inner periphery of the wells of the microwell plate by, at most, 5%.

14. The system of any one of claims 11 to 13, further comprising a pair of electrodes (1502) for stimulating muscle tissue (108).

15. A method of manufacturing a device (100) for testing in vitro muscle tissue (108), the method comprising: injection molding the device (100) using a mold which defines:

> a base section (104);
> two pillars (102) each protruding from the base section (104); and
> a wall section (106) with an inner wall surface surrounding the pillars (102), and an outer wall surface, wherein the wall section (106) comprises at least two rib elements (1002) extending outwardly from the outer wall surface to an outer periphery of the device (100).

a)

106    102    108    102    106

100

104

b)

106    102    108    102    106

100

104

c)

108

102    102    100

FIG.1

FIG.2

FIG.3

FIG.4

FIG.5

FIG.6

FIG.7

FIG.8

a) 100

b) 100

FIG.9

100

1002

1002

106

1002

104

1002

FIG.10

100

1002

102

102

106

104

1002

FIG.11

1202

100

FIG.12

FIG.13

100

FIG.14

FIG.15

FIG.16

FIG.17

FIG.18

Europäisches
Patentamt

European
Patent Office

Office européen
des brevets

# EUROPEAN SEARCH REPORT

Application Number

EP 20 20 9706

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2019/316068 A1 (CHEN CHRISTOPHER S [US] ET AL) 17 October 2019 (2019-10-17) * paragraph [0132] - paragraph [0138]; figures 12, 13A-13D * | 1-15 | INV. G01N33/483 |
| Y | US 2007/212775 A1 (COHEN DAVID A [US] ET AL) 13 September 2007 (2007-09-13) * paragraph [0054] * * paragraph [0058] * * paragraph [0078] - paragraph [0080] * * figures 1,2 * | 1-15 | |
| A | US 2014/363610 A1 (SAMEOTO DANIEL ELLIOT [CA]) 11 December 2014 (2014-12-11) * abstract * | 7 | |
| A | US 2014/220555 A1 (CHEN CHRISTOPHER S [US] ET AL) 7 August 2014 (2014-08-07) * paragraph [0021] * * paragraph [0046] * * paragraph [0056] - paragraph [0057] * * paragraph [0073] * * figures 7,15 * | 1-15 | |
| A | XIAO FAN ET AL: "Plasmonic micropillars for precision cell force measurement across a large field-of-view", APPLIED PHYSICS LETTERS, A I P PUBLISHING LLC, US, vol. 112, no. 3, 17 January 2018 (2018-01-17), XP012225574, ISSN: 0003-6951, DOI: 10.1063/1.5005525 [retrieved on 2018-01-17] * figure 1 * | 1,15 | |

TECHNICAL FIELDS SEARCHED (IPC)

G01N

-/--

The present search report has been drawn up for all claims

| Place of search | Date of completion of the search | Examiner |
|---|---|---|
| The Hague | 23 April 2021 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 006 543 A1

**EUROPEAN SEARCH REPORT**

Application Number

EP 20 20 9706

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Nathan J. Sniadecki ET AL: "Microfabricated Silicone Elastomeric Post Arrays for Measuring Traction Forces of Adherent Cells" In: "Cytometry: Part B", 1 January 2007 (2007-01-01), San Diego [u.a.] : Acad. Press, US, XP055227530, ISSN: 0091-679X vol. 83, pages 313-328, DOI: 10.1016/S0091-679X(07)83013-5, * figure 2 * | 1,15 | |
| A | BEUSSMAN KEVIN M ET AL: "Micropost arrays for measuring stem cell-derived cardiomyocyte contractility", METHODS, ACADEMIC PRESS, NL, vol. 94, 3 September 2015 (2015-09-03), pages 43-50, XP029422114, ISSN: 1046-2023, DOI: 10.1016/J.YMETH.2015.09.005 * figure 4 * | 1,15 | |
| A | W. R. LEGANT ET AL: "Microfabricated tissue gauges to measure and manipulate forces from 3D microtissues", PROCEEDINGS OF THE NATIONAL ACADEMY OF SCIENCES, vol. 106, no. 25, 23 June 2009 (2009-06-23), pages 10097-10102, XP055149619, ISSN: 0027-8424, DOI: 10.1073/pnas.0900174106 * the whole document * | 1-15 | TECHNICAL FIELDS SEARCHED (IPC) |

-/--

The present search report has been drawn up for all claims

| Place of search | Date of completion of the search | Examiner |
|---|---|---|
| The Hague | 23 April 2021 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches Patentamt

European Patent Office

Office européen des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DOSTANIC MILICA ET AL: "A Miniaturized EHT Platform for Accurate Measurements of Tissue Contractile Properties", JOURNAL OF MICROELECTROMECHANICAL SYSTEMS, IEEE SERVICE CENTER, US, vol. 29, no. 5, 1 October 2020 (2020-10-01), pages 881-887, XP011813319, ISSN: 1057-7157, DOI: 10.1109/JMEMS.2020.3011196 [retrieved on 2020-10-05] * the whole document * | 1-15 | |
| A | FLORIAN WEINBERGER ET AL: "Engineering Cardiac Muscle Tissue : A Maturating Field of Research", CIRCULATION RESEARCH, vol. 120, no. 9, 28 April 2017 (2017-04-28), pages 1487-1500, XP055615379, US ISSN: 0009-7330, DOI: 10.1161/CIRCRESAHA.117.310738 * the whole document * | 1-15 | |

TECHNICAL FIELDS SEARCHED (IPC)

The present search report has been drawn up for all claims

| Place of search | Date of completion of the search | Examiner |
|---|---|---|
| The Hague | 23 April 2021 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 20 9706

This annex lists the patent family members relating to the patent documents cited in the above-mentioned European search report.
The members are as contained in the European Patent Office EDP file on
The European Patent Office is in no way liable for these particulars which are merely given for the purpose of information.

23-04-2021

| Patent document cited in search report | | Publication date | Patent family member(s) | | Publication date |
|---|---|---|---|---|---|
| US 2019316068 | A1 | 17-10-2019 | US 2019316068 A1<br>WO 2019200042 A1 | | 17-10-2019<br>17-10-2019 |
| US 2007212775 | A1 | 13-09-2007 | NONE | | |
| US 2014363610 | A1 | 11-12-2014 | NONE | | |
| US 2014220555 | A1 | 07-08-2014 | EP 2766473 A1<br>US 2014220555 A1<br>WO 2013056019 A1 | | 20-08-2014<br>07-08-2014<br>18-04-2013 |

EPO FORM P0459

For more details about this annex : see Official Journal of the European Patent Office, No. 12/82